# EUROPEAN PATENT APPLICATION

(11) **EP 1 346 729 A1**
(43) Date of publication of application: **24.09.2003**
(21) Application number: 02076146.6
(22) Date of filing: 19.03.2002
(51) Int. Cl.: A61K 47/48

(54) **Targeting of myocardial angiogenesis through CD13/APN**

(71) Applicant: Cardiovascular Research Institute Maastricht, 6229 ER Maastricht (NL); St. Jude's Chrildren's Research Hospital, Memphis, TN 38105 (US)
(72) Inventor: DeMuinck, Ebo D., Hanover, NH 03755 (US); Shapiro, Linda H., Middlebury, CT 06762 (US)
(74) Representative: van Westenbrugge, Andries

(57) **Abstract**

The present invention relates to conjugates comprising a CD13/APN homing molecule that is conjugated with a moiety for treating or diagnosing a cardiovascular disease. The invention further relates to methods in which these conjugates are used to target therapeutic agents or imaging molecules to the cardiovascular system. In particular the invention relates to conjugates comprising a CD13/APN homing molecule that is conjugated with angiogenesis-promoting factors for use in the treatment of ischemic myocardium or extremities. The invention also relates to conjugates comprising a CD13/APN homing molecule that is conjugated with a moiety that favourably alters wound healing after percutaneous arterial intervention. The invention further relates to conjugates comprising a CD13/APN homing molecule that is conjugated with a moiety that favourably alters atherosclerotic plaques, such as an angiogenesis inhibitor, for use in stabilising or reducing the size of atherosclerotic plaques. Finally, the invention relates to conjugates comprising a CD13/APN homing molecule that is conjugated with an imaging moiety for use in diagnostic methods comprising vascular imaging.

## Description

### Field of the invention

The present invention relates to methods of targeting therapeutic agents and imaging molecules to the cardiovascular system. In particular the invention relates to the use of specific peptides that home to the CD13/APN molecule as expressed in angiogenic vessels in cardiovascular diseases.

### Background of the invention

Many cell surface glycoproteins expressed during specific stages of lymphoid and myeloid cell development were initially characterised on the basis of their reactivity with monoclonal antibodies. Subsequent molecular cloning of the gene encoding one of these hematopoietic differentiation antigens, CD 13 (cluster of differentiation 13), identified it as the membrane bound metalloproteinase, aminopeptidase N (APN) (Look et al., 1989, J. Clin. Invest. 83: 1299-1306). Recently, CD13/APN has been shown to become activated by angiogenic signals and to be essential for capillary tube formation (Bhagwat et al., 2001, Blood 97: 652-659). Studies designed to identify unique peptides that home specifically to solid tumours in murine breast carcinoma models revealed that the NGR motif (i.e. the amino acid sequence asparagine-glycine-arginine) binds strictly to the endothelium of angiogenic blood vessels in tumours (Pasqualini et al., 1995, J. Cell Biol. 130: 1189-1196).

Further investigation identified CD13/APN as the principal receptor for this peptide motif and demonstrated that this protein is expressed exclusively on the endothelial cells of angiogenic but not normal vasculature, thereby explaining the tumour-specific nature of the NGR peptide (Pasqualini et al., 2000, Cancer Res. 60: 722-727). Furthermore, it has been demonstrated that NGR peptides can be used for targeted drug delivery to tumour vasculature. Studies in murine cancer models with doxorubicin and pro-apoptotic peptides coupled to NGR peptides showed improved survival and reduction in tumour mass, thereby demonstrating the therapeutic potential NGR peptides coupled to therapeutic agents (US 6,180,084; Ellerby et al., 1999, Nature Med. 5: 1032-1038).

The art, however, does not disclose the site-specific expression of CD13/APN in the angiogenic peri-infarct zone after myocardial infarction, nor does the art disclose the presence of CD13/APN in the vasculature of complex atherosclerotic plaques. Rather, US 6,180,084 discloses that although CD13/APN is expressed outside the vascular system, CD13/APN having immunoreactivity with an anti-CD13 antibody is only exposed to the circulation in tumour vessels.

### Description of the invention

The present invention is based on the surprising discovery that CD13/APN is site-specifically expressed in angiogenic areas in ischemic myocardium, e.g. in the peri-infarct zone after myocardial infarction, as well as in the vasculature of complex atherosclerotic plaques. The presence of CD13/APN in these specific areas of cardiovascular neo-vascularisation reveals the potential for using CD13/APN homing agents as a means to deliver therapeutic agents to angiogenic myocardium and to angiogenic, i.e. rupture prone, atherosclerotic plaques. Likewise, CD13/APN homing agents may be used as a means to deliver imaging molecules or structures to these areas. This will allow for non-invasive imaging of myocardial angiogenesis and will facilitate monitoring of the development of neo-vascularisation over time, e.g. in response to angiogenic growth factor therapy. Likewise, this visualisation technique may be applied to establish the presence of angiogenic, i.e. rupture prone atherosclerotic plaques and will allow for monitoring the status of such plaques over time. Thus, patients at risk of plaque rupture may be identified at an early stage, treated with therapeutic agents that home to CD13/APN and the therapeutic effect may be monitored non-invasively with CD13/APN targeted molecules.

Thus, in its broadest sense the present invention relates to a conjugate comprising a CD13/APN homing molecule that is conjugated with a moiety for treating or diagnosing a (cardio)vascular disease, to methods in which these conjugates are used for treating or diagnosing a (cardio)vascular disease and to use of these conjugates in the preparation of a medicament for treating or diagnosing a (cardio)vascular disease.

### CD13/APN homing agents

The present invention is based on the site-specific delivery of useful moieties to angiogenic cardiovascular areas by using CD13/APN as a target molecule. CD13/APN is a highly conserved transmembrane glycoprotein of about 150 kDa that is incorporated into the cell membrane through an N-terminal hydrophobic segment (Look et al., 1989, supra; Xu et al., 1997, Exp. Hematol. 25:521-529). The large extracellular carboxy-terminal domain contains a pentapeptide that is characteristic of many zinc-dependent metalloproteases (Look et al., supra, 1989). Homologues of CD13/APN from several different species are well conserved (Look et al., supra, 1989; Xu et al., supra, 1997; Turner et al., in Mammalian Ectoenzymes, Kenny and Turner, eds., Elsevier Scientific Publishing Co., Amsterdam, p. 211 (1987)).

The term "CD13/APN" is herein understood to mean a polypeptide with the amino acid sequence that is substantially similar to the amino acid sequence of SEQ ID NO: 1. The term "CD13/APN" thus comprises the human CD13/APN as well as its non-human mammalian homologues and includes allelic forms and muteins of these polypeptides comprising one or more amino acid substitutions, deletions and/or insertions. The term "CD13/APN" as used herein thus comprises polypeptides having at least 90% amino acid identity with the amino acid sequence of SEQ ID NO: 1 and preferably containing the above-mentioned pentapeptide that is characteristic of many zinc-dependent metalloproteases.

The amino acid identity between a polypeptide comprised in the term "CD13/APN" and SEQ ID NO: 1 may be readily calculated by known methods, including but not limited to those described in (Computational Molecular Biology, Lesk, A. M., ed., Oxford University Press, New York, 1988; Biocomputing: Infomatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988). Preferred methods to determine identity are designed to give the largest match between the sequences tested. Methods to determine identity are codified in publicly available computer programs. Preferred computer program methods to determine identity and similarity between two sequences include, but are not limited to, the GCG program package (Devereux, J., et al., Nucleic Acids Research 12 (1): 387 (1984), BestFit, BLASTP, BLASTN, and FASTA (Altschul, S. F. et al., J. Mol. Biol. 215:403-410 (1990)). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990)). The well-known Smith Waterman algorithm may also be used to determine identity. Preferred parameters for polypeptide sequence comparison include the following: 1) Algorithm: Needleman and Wunsch, J. Mol. Biol. 48:443-453 (1970) Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for peptide comparisons (along with no penalty for end gaps).

CD13/APN may be further defined by the fact the enzymatic activity of cell-surface CD13/APN can be blocked by bestatin, o-phenanthroline, actinonin and antibodies that block CD13/APN activity (2-M7 and R3-63, A. Strhyn, State Serum Institute, Copenhagen, Denmark). In addition, CD13/APN may be defined by its capability to bind specifically to peptide containing the NGR motif.

CD13/APN is expressed in many different cell types, including e.g. normal and malignant cells of the myeloid lineage, as well as in many endothelial and tumour cell types and CD13/APN can function differently depending on its location (reviewed e.g. in US 6,180,084). Most relevant in the context of the present invention, however, is the expression of CD13/APN on the cell membranes of endothelial cells of angiogenic vasculature but not of normal vasculature (Pasqualini et al., 2000, supra). Functional expression of CD13/APN in angiogenic vasculature appears to be required for vascular endothelial cell capillary tube formation and migration, but not for proliferation (Bhagwat et al., 2001, supra).

In the present invention a CD13/APN homing molecule is applied to target of useful entities to angiogenic cardiovascular areas. A "CD13/APN homing molecule" is herein defined as a molecule that specifically binds to CD13/APN. As used herein, the term "specific binding" means binding that is measurably different from a non-specific interaction. Specific binding can be measured, for example, by determining binding of a molecule compared to binding of a control molecule, which generally is a molecule of similar structure that does not have binding activity, for example, a peptide of similar size that lacks a specific binding sequence (e.g. NGR in the case of CD13/APN). Specific binding is present if the molecule has measurably higher affinity for the CD13/APN than the control molecule. Specificity of binding can be determined, for example, by competition with a control molecule that is known to bind to a target. E.g., specific binding of an NGR peptide can be demonstrated by competing for binding with the same NGR peptide or a different peptide containing an NGR motif. In this case, specific binding is indicated if the binding of a molecule is competitively inhibited by the second NGR containing peptide.

The term "specific binding," as used herein, includes both low and high affinity specific binding. Specific binding can be exhibited, e.g., by a low affinity CD13/APN homing molecule having a Kd of at least about 10⁻⁴ M. E.g., if CD13/APN has more than one binding site for a CD13/APN homing molecule, a CD13/APN homing molecule having low affinity can be useful for targeting angiogenic vasculature. Specific binding also can be exhibited by a high affinity CD13/APN homing molecule, for example, a CD13/APN homing molecule having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater. Both low and high affinity CD13/APN homing molecules are useful for targeting angiogenic vasculature.

A CD13/APN homing molecule for use in the present invention is capable of specific binding to CD13/APN and is suitable for conjugation to a useful moiety, which may be a therapeutic molecule moiety or an imaging molecule or structure. A preferred CD13/APN homing molecule for use in the present invention is a peptide containing the NGR motif, i.e. the amino acid sequence asparagine-glycine-arginine. In principle any peptide, peptide, polypeptide or protein containing an NGR motif may be used as CD13/APN homing molecule. NGR containing oligopeptides, polypeptides and proteins will collectively be referred to as (NGR containing) peptides. For convenience and economics, NGR containing peptides of limited length (i.e. oligopeptides) are preferred. NGR containing peptides preferably have a length of less than 30, 25, 20, 15 or 10 amino acids. The minimal length of the peptide is determined by the NGR sequence, however peptide with a length of at least 5, 6, 7, 8, or 9 amino acids are preferred in view of the fact that the additional non-NGR motif amino acids may be required for conjugation of the peptide without interfering with the NGR motif's capability of specific binding to CD13/APN.

The position of the NGR motif within the peptide sequence may also be optimised with respect to avoiding conjugation of the peptide and possible interference with its binding to CD13/APN. E.g. in the case of carbodiimide conjugation, which use a carboxyl group on the peptide for conjugation, the NGR motif is preferably positioned towards the N-terminus of the peptide and amino acids with carboxyl groups, such as glutamate and aspartate, are preferably not present in close proximity to the NGR motif within the peptide's sequence. Several methods for conjugation of peptides to substrates or available in the art (see herein below) and based on the above guidelines the skilled person is capable of designing NGR containing peptide sequence that are optimised with respect to avoid interference of conjugation with CD13/APN binding.

The amino acid sequence of the NGR containing peptide or other CD13/APN homing peptides preferably also does not contain amino acid motifs that cause the peptide to (specifically) bind to non-CD13/APN targets. Specifically, amino acid motifs that bind to non-CD13/APN targets exposed in or to circulating blood are to be avoided from CD13/APN homing peptides. Several such, usually short, amino acid motifs are known in the art, such as e.g. the RGD sequence that binds to integrins.

Several useful example of NGR containing peptides that haven proven their use as CD13/APN homing molecules are described in US 6,180,084 and include peptides with amino acid sequences, CNGRCVSGCAGRC (SEQ ID NO: 2), NGRAHA (SEQ ID NO: 3), CVLNGRMEC (SEQ ID NO: 4) or CNGRC (SEQ ID NO: 5).

Preferred peptides contain cysteine residues on either side of the NGR motif that may be obtained in cyclic form, i.e. with a disulphide bond between the two cysteine residues. Most preferred are such peptides with each of the cysteine residue is present in a terminal position. Such peptides include e.g. CNGRCVSGCAGRC (SEQ ID NO: 2), CVLNGRMEC (SEQ ID NO: 4) or CNGRC (SEQ ID NO: 5). Any of these above peptides may likewise be applied as CD13/APN homing molecules for conjugation in the present invention.

In addition to the NGR containing peptides other peptide sequences, peptide-like molecules (referred to as peptidomimetics) or non-peptide molecules that specifically bind to CD13/APN and that may be applied as CD13/APN homing molecules may be identified using methods known in the art per se, as e.g. described in detail in US 6,180,084 which incorporated herein by reference. Such methods include e.g. screening libraries of peptidomimetics, peptides, DNA or cDNA expression libraries, combinatorial chemistry and, particularly useful, phage display libraries. These libraries may be screened for CD13/APN homing molecules by contacting the libraries with substantially purified CD13/APN, fragments thereof or structural analogues thereof.

CD13/APN homing peptides may be synthesised by standard methods of solid phase chemistry known to those of ordinary skill in the art. E.g. CD13/APN homing peptides may be synthesised by solid phase chemistry techniques following the procedures described by Steward and Young (Steward, J. M. and Young, J. D., Solid Phase Peptide Synthesis, 2nd Ed., Pierce Chemical Company, Rockford, I11., (1984) using an Applied Biosystem synthesiser. Similarly, multiple peptide fragments may be synthesised then linked together to form larger fragments. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, Vol. 1, Academic Press (New York). In general, these methods comprise the sequential addition of one or more amino acids or suitably protected amino acids to a growing peptide chain. Normally, either the amino or carboxyl group of the first amino acid is protected by a suitable protecting group. The protected or derivatised amino acid is then either attached to an inert solid support or utilised in solution by adding the next amino acid in the sequence having the complimentary (amino or carboxyl) group suitably protected and under conditions suitable for forming the amide linkage. The protecting group is then removed from this newly added amino acid residue and the next amino acid (suitably protected) is added, and so forth. After all the desired amino acids have been linked in the proper sequence, any remaining protecting groups (and any solid support) are removed sequentially or concurrently to afford the final (poly)peptide.

CD13/APN homing peptides may not be particularly useful when administered orally because they can be degraded in the digestive tract. However, methods for chemically modifying peptides to render them less susceptible to degradation by endogenous proteases or more absorbable through the alimentary tract are well known (see, e.g., Blondelle et al., 1995, Trends Anal. Chem. 14: 83-92; Ecker and Crooke, 1995, Biotechnology 13: 351-360; Goodman and Ro, Peptidomimetics for Drug Design, in: "Burger's Medicinal Chemistry and Drug Discovery" Vol. 1 (ed. M. E. Wolff; John Wiley & Sons 1995), pages 803-861). Alternatively D-amino acids may be incorporated into CD13/APN homing peptides in order to increase their proteolytic resistance.

Further molecules that may be applied as CD13/APN homing molecule for conjugation in the present invention include CD13/APN substrate analogues and/or aminopeptidase inhibitors such as bestatin, O-phenanthroline, actinonin, amastatin, 2,2'-dipyridyl or fumagillin.

### Conjugates and methods for conjugation

A "conjugate" is herein defined as consisting of two entities that are covalently coupled together. In the context of the present invention the first entity will usually be a CD13/APN homing molecule as herein defined above, whereas the second entity may be a therapeutic or diagnostic moiety, such as a molecule or structure, for use in the treatment or diagnosis of cardiovascular disease. Such therapeutic or diagnostic moieties may be as herein defined below, including e.g. an angiogenesis-promoting molecule, an angiogenesis-inhibiting molecule, or an imaging molecule or structure.

A large variety of methods for conjugation of CD13/APN homing molecules with therapeutic or diagnostic moieties are known in the art. Such methods are e.g. described by Hermanson (1996, Bioconjugate Techniques, Academic Press), in U.S. 6,180,084 and U.S. 6,264,914 and include e.g. methods used to link haptens to carriers proteins as routinely used in applied immunology (see Harlow and Lane, 1988, "Antibodies: A laboratory manual", Cold Spring Harbor Laboratory Press, Cold Spring Harbor, NY). It is recognised that, in some cases, a CD13/APN homing molecule or a therapeutic or diagnostic moiety may lose efficacy or functionality upon conjugation depending, e.g., on the conjugation procedure or the chemical group utilised therein. However, given the large variety of methods for conjugation the skilled person is able to find a conjugation method that does not or least affects the efficacy or functionality of the entities to be conjugated.

Suitable methods for conjugation of a CD13/APN homing molecule with a therapeutic or diagnostic moiety include e.g. carbodiimide conjugation (Bauminger and Wilchek, 1980, Meth. Enzymol. 70: 151-159). Alternatively, a moiety can be coupled to a CD13/APN homing molecule as described by Nagy et al., Proc. Natl. Acad. Sci. USA 93:7269-7273 (1996); and Nagy et al., Proc. Natl. Acad. Sci. USA 95:1794-1799 (1998), each of which is incorporated herein by reference. Another method for conjugating that may suitable be used are e.g. sodium periodate oxidation followed by reductive alkylation of appropriate reactants and glutaraldehyde crosslinking.

A particularly advantageous method of conjugation may be applied when both the CD13/APN homing molecule and the therapeutic moiety are (poly)peptides. In such instances the two entities may be synthesised as a single (poly)peptide chain comprising the amino acid sequences of both the CD13/APN homing peptide and the therapeutic peptide. When the sum of the amino acid sequences of the CD13/APN homing peptide and the therapeutic peptide does not exceed 50, 80 or 100 amino acids the conjugate may be synthesised by solid phase peptide synthesis as herein described above. Alternatively, when the sum of the amino acid sequences is larger the single (poly)peptide chain comprising the CD13/APN homing peptide and the therapeutic peptide may be produced by recombinant expression techniques as outlined herein below. In such instances e.g. the two nucleic acid sequences encoding each the CD13/APN homing peptide and the therapeutic peptide may be operably linked in frame to form a single open reading frame. The nucleic acid sequence containing the single open reading frame may then be inserted in a suitable expression vector for expression in a suitable host from which the conjugate may then be recovered and optionally further purified. In these methods, CD13/APN homing peptides may be placed on either or both ends of the therapeutic peptide, or may be inserted within the amino acid sequence of the therapeutic peptide in one or more positions that do not disturb the function or efficacy of the respective peptides. Using routine methods the skilled person can establish the optimal position of the CD13/APN homing peptide(s) with respect to the therapeutic peptide.

### Therapeutic angiogenesis

Therapeutic myocardial angiogenesis has been shown to lead to improved myocardial perfusion in animal models (Li et al., 2000, Nature Med. 6: 49-50) and several clinical trails of myocardial angiogenesis by gene therapy are ongoing or have been completed (Ylä-Hettualla et al., 2000, Lancet 355: 213-222). The delivery of angiogenic growth factors should, however, be simple and target specific. Since no clear means of maximising myocardial distribution and retention exist, most of these trails have used catheter based or surgical techniques rather than intravenous administration to achieve maximum myocardial exposure to growth factor. In one aspect, the present invention addresses these problems by using a CD13/APN homing molecule to deliver angiogenesis-promoting molecules to critical areas in myocardial disease.

In a first aspect, the invention, therefore, relates to a conjugate comprising a CD13/APN homing molecule that is conjugated with an angiogenesis-promoting molecule. The CD13/APN homing molecule is a homing molecule as herein defined above and may be conjugated to the angiogenesis-promoting molecule by any of the means as described herein above. The angiogenesis-promoting molecule preferably is an angiogenic growth factor. Alternatively, the angiogenesis-promoting molecule is an inhibitor of molecules that are anti-angiogenic. The angiogenic growth factor preferably is a peptide, wherein the term peptide is understood to include (larger) polypeptides. Preferred angiogenic growth factors are VEGF (vascular endothelial growth factor) and P1GF (placental growth factor), either has monomer, homodimer and preferably as a VEGF/P1GF heterodimer, bFGF (basic fibroblast growth factor), the macrophage-derived peptide PR39, its truncated version PR11, CD154, of which preferably the extracellular domain in soluble form, platelet derived growth factor (PDGF), hepatocyte growth factor (HGF) or scatter factor. The growth factors are preferably obtained in recombinant form through expression in a suitable host organism or cell. The DNA and amino acid sequence of VEGF and methods for producing recombinant VEGF are e.g. known from U.S. 5,994,300. The DNA and amino acid sequence of bFGF and methods for producing recombinant bFGF are e.g. known from WO 87/01728. The DNA and amino acid sequence of CD154 (also known as CD40-ligand) and methods for producing recombinant CD154 are e.g. known from U.S. 5,961,974. The P1GF DNA and amino acid sequence can be found in the Genbank database under accession no. XM_040407.

When the growth factors are produced by recombinant means, they may be suitably conjugated to a CD13/APN homing peptide by operably linking the respective nucleic acid sequences encoding the CD13/APN homing peptide and the growth factor, such that the conjugate can be expressed as a single polypeptide chain comprising the amino acid sequences of both the growth factor and the CD13/APN homing peptide. Methods for the recombinant production of (poly)peptides and modification and/or construction of nucleic acids are as described herein below. Alternatively, the (recombinant) growth factors may be conjugated by conventional chemical means as herein described above.

Peptide growth factors like PR39 are preferably obtained through chemical synthesis as herein described above for the CD13/APN homing peptides.

In an alternative embodiment, the angiogenesis-promoting molecule in the conjugate is a nucleic acid capable of expressing an angiogenic growth factor. The nucleic acid preferably is a gene therapy vector that comprises a sequence encoding an angiogenic growth factor operably linked to appropriate expression signals. The CD13/APN homing molecule may be directly conjugated to a nucleic acid comprising the sequence coding for the angiogenic factor, such as e.g. a plasmid. Alternatively, the CD13/APN homing molecule may be conjugated to or expressed on a viral capsid protein (or another protein expressed at the surface of a virion) of a virus used as a gene therapy vector that carries sequence coding for the angiogenic factor.

An advantage of targeting angiogenic growth factors such as P1GF, VEGF, bFGF and CD154 to CD13/APN resides in the synergy between these growth factors and CD13/APN itself on angiogenesis. This results in two strong angiogenesis enhancing effects. The angiogenic growth factors cause upregulation of CD13/APN, resulting in two angiogenesis enhancing effects. First, upregulation of CD13/APN in the angiogenic vasculature, provides a larger target density upon repeated administration of the CD13/APN targeted growth factor. Second, the upregulation of CD13/APN will further enhance angiogenesis because CD13/APN is not a passive target for homing molecules but also a regulator of angiogenesis. Hence upregulation of CD13/APN by growth factors linked to CD13/APN homing molecules will achieve further enhancement of angiogenesis through presenting a larger target area upon repeated administration as well as through promoting of angiogenesis.

A second aspect of the invention relates to a composition, preferably a pharmaceutical composition comprising the conjugates of the CD13/APN homing molecule and the angiogenesis-promoting molecule. The composition may comprise one such conjugate or may comprise combinations of different angiogenesis promoting conjugates that may act in synergy. The composition may in addition comprise angiogenic growth factors as such. A particularly preferred composition comprise a conjugate of the CD13/APN homing molecule and (recombinant) P1GF and in addition comprises VEGF as such, whereby P1GF and VEGF are allowed to form the synergistic P1FG/VEGF heterodimer which is more potent than either homodimer. Also, both P1GF and VEGF promote upregulation of CD13/APN expression resulting in the synergistic effects on angiogenesis described above. Likewise, the composition may comprise P1GF as such and VEGF in conjugated form. The pharmaceutical composition comprises at least one purified conjugate of the CD13/APN homing molecule and the angiogenesis-promoting molecule and a pharmaceutical carrier. The pharmaceutical composition is further as herein described below. A preferred pharmaceutical composition is suitable for parenteral, or more preferably intravenous or intraarterial administration.

In a third aspect the invention relates to a method for treating an ischemic myocardium, i.e. a myocardium with at least a part or zone that is ischemic. The method comprises administering any of the pharmaceutical compositions comprising the conjugate of the CD13/APN homing molecule and the angiogenesis-promoting molecule as defined above, to a subject suffering from an ischemic myocardium. The subject may be a subject suffering from impaired myocardial perfusion resulting from atherosclerotic narrowing of one or more coronary arteries. The method may be a method for treating a chronic ischemic myocardium, or the method may be a method for treating an ischemic myocardium resulting from a myocardial infarction. Preferably the method is a method for improving perfusion of the ischemic myocardium, a method for reducing the size of the ischemic zone in the ischemic myocardium, a method for reducing the amount of myocardial tissue loss in an ischemic myocardium, or a method for preventing further tissue loss in an ischemic myocardium, e.g. after a myocardial infarction or in a chronic ischemic myocardium.

In a fourth aspect the invention relates to a method for treating an ischemic extremity (upper or lower). The method comprises administering any of the pharmaceutical compositions comprising the conjugate of the CD13/APN homing molecule and the angiogenesis-promoting molecule as defined above, to a subject suffering from an ischemic extremity. Preferably the method is a method for improving perfusion of the ischemic extremity, a method for reducing the size of the ischemic zone in the ischemic extremity, a method for reducing the amount of tissue loss in an ischemic extremity, or a method for preventing further tissue loss in an ischemic extremity, e.g. after an arterial occlusion or in a chronic ischemic extremity.

In a fifth aspect the invention relates to a method for improving the recovery of a subject after percutaneous coronary or peripheral intervention, wherein the method comprises administering any of the pharmaceutical compositions comprising the conjugate of the CD13/APN homing molecule and the angiogenesis-promoting molecule, to a subject having undergone percutaneous coronary or peripheral intervention. Preferably, the method is a method for accelerating wound healing after percutaneous coronary intervention. Preferably such a method is a method for preventing or reducing neointimal thickening of treated arterial segments.

Preferably, in any of the above methods the pharmaceutical composition comprising the conjugate of the CD13/APN homing molecule and the angiogenesis-promoting molecule is administered via an intravenous or intraarterial route. In any of these methods, the pharmaceutical composition may be administered by (continuous) infusion or by bolus injection(s), or by combinations thereof. Preferably, in any of the above methods (the pharmaceutical composition comprising) the conjugate of the CD13/APN homing molecule and the angiogenesis-promoting molecule is administered in a therapeutically effective amount, preferably such that the stated therapeutic effects are achieved. The dose at which the therapeutic effects are achieved is anticipated to be lower than the dose for systemic, non-targeted administration because of the target specificity of NGR/angiogenic conjugates that home to CD13/APN.

In a sixth aspect the invention relates to use of a conjugate of the CD13/APN homing molecule and the angiogenesis-promoting molecule as defined above, in the manufacture of a medicament or a pharmaceutical composition for use in any of the angiogenesis-promoting therapeutic methods described above. The pharmaceutical composition preferably is suitable for administration via an intravenous or intraarterial route.

An additional advantage of angiogenic therapy based on homing of angiogenesis-promoting molecules to CD13/APN, lies in the synergistic effect on angiogenesis of the angiogenesis-promoting molecule on the one hand, and CD13/APN on the other hand. Angiogenesis-promoting molecules stimulate expression of CD13/APN, which in itself is a positive regulator of angiogenesis. Therefore, increased expression of CD13/APN further promotes angiogenesis and at the same time makes available more CD13/APN targets for homing of the conjugates with the angiogenesis-promoting molecules.

### Inhibition of angiogenesis in (cardio)vascular disease

The vasa vasorum consist of blood vessels that vary in size from vessels with single or multiple smooth cell layers to simple endothelial channels. Atherosclerotic plaque growth is associated with proliferation of vasa vasorum. Moulton et al. (1999, Circulation 99: 1726-1732) recently showed that plaque area can be reduced by administration of angiogenesis inhibitors. The major cause of morbidity and mortality related to atherosclerotic cardiovascular disease is plaque rupture with subsequent intra-coronary thrombosis and there are several lines of evidence that link plaque rupture to increased angiogenic activity and hemorrhage within the plaque. Thus it is a further object of the present invention to use a CD13/APN homing molecule to deliver therapeutic moieties to atherosclerotic plaques in the prevention and treatment of plaque rupture.

In a seventh aspect, the invention, therefore, relates to a conjugate comprising a CD13/APN homing molecule that is conjugated with a moiety that favourably alters atherosclerotic plaques. This favourable modification includes stabilising plaques and reducing their size by reducing plaque vascularisation with anti-angiogenic agents; improving endothelial function with HMG-coreductase inhibitors, e-NOS or i-NOS gene transfer, anti-oxidants, 5-methyltetrahydrofolate; interrupting inflammatory signalling e.g. through inhibition of CD40-CD154 signalling with CD154-antibody. The CD13/APN homing molecule is a homing molecule as herein defined above and may be conjugated to the plaque stabilising/reducing moiety by any of the means as described herein above. Preferably the moiety is a moiety or molecule that stabilises rupture prone atherosclerotic plaques. A preferred moiety for stabilising or reducing atherosclerotic plaques is an angiogenesis inhibitor.

Many different angiogenesis inhibitors as well as methods for their production and use are known in the art. Any of these angiogenesis inhibitors may be applied in the present invention for conjugation to a CD13/APN homing molecule. Such angiogenesis inhibitors include e.g. anti-angiogenic peptides such as endostatin (U.S. 5,854,205), bestatin, TNP-470 (Teicher et al., 1994, Int. J. Cancer 57: 920-925), angiostatin (WO 95/29242) and the kringle 5 domain fragment from (human) plasminogen (U.S. 5,801,146); thrombospondin 1 and 2; (monoclonal) antibodies against angiogenic growth factors or receptors therefor, including fragments of such antibodies such as Fab fragments; (small) organic molecules that act as angiogenesis inhibitors including e.g. suramin, 3-(3-substituted-1,2,4-thiadiazol-5-yl)pyridazine derivatives (U.S. 6,265,407), thalidomide (U.S. 6,235,756) and cytochalasin and iso-indolinone derivatives (U.S. 5,994,388). Preferred angiogenesis inhibitors are anti-angiogenic peptides of which endostatin and TNP-470 are most preferred.

An eighth aspect of the invention relates to a composition, preferably a pharmaceutical composition comprising the conjugates of the CD13/APN homing molecule and the moiety that stabilises or reduces atherosclerotic plaques, such as the angiogenesis inhibitors. The composition may comprise one such conjugate or may comprise combinations of different angiogenesis inhibiting conjugates that may act in synergy. The composition may in addition comprise molecules that stabilises or reduces atherosclerotic plaques (such as angiogenesis inhibitors) as such. The pharmaceutical composition comprises at least one purified conjugate of the CD13/APN homing molecule and the moiety that stabilises or reduces atherosclerotic plaques and a pharmaceutical carrier. The pharmaceutical composition is further as herein described below. A preferred pharmaceutical composition is suitable for parenteral, or more preferably intravenous or intraarterial administration.
In an ninth aspect the invention relates to a method for the treatment of an atherosclerotic plaque, wherein the method comprises administering the pharmaceutical compositions comprising the conjugate of the CD13/APN homing molecule and the moiety that stabilises or reduces atherosclerotic plaques as defined above, to a subject having (or suspected of having) an atherosclerotic plaque. The atherosclerotic plaque preferably is a rupture prone atherosclerotic plaque. The method preferably is a method for stabilising the atherosclerotic plaque or for preventing rupture of the atherosclerotic plaque. More preferably the method is a method for reducing the size of the atherosclerotic plaque. Preferably, in any of the above methods the pharmaceutical composition comprising the conjugates of the invention is administered via an intravenous or intraarterial route. In any of these methods, the pharmaceutical composition may be administered by (continuous) infusion or by bolus injection(s), or by combinations thereof. Preferably, in any of the above methods (the pharmaceutical composition comprising) the conjugate of the CD13/APN homing molecule and the moiety that stabilises or reduces atherosclerotic plaques as defined above is administered in a therapeutically effective amount, preferably such that the stated therapeutic effects are achieved. The dose at which the therapeutic effects are achieved is anticipated to be lower than the dose for systemic, non-targeted administration because of the target specificity of NGR/angiogenic conjugates that home to CD13/APN.

In a tenth aspect the invention relates to use of a conjugate of the CD13/APN homing molecule and the moiety that stabilises or reduces atherosclerotic plaques as defined above, in the manufacture of a medicament or a pharmaceutical composition for use in any of the treatments of atherosclerotic plaques described above. The pharmaceutical composition preferably is suitable for administration via an intravenous or intraarterial route.

### Vascular imaging

Magnetic resonance (MR) imaging has enormous potential in assessing myocardial and vascular structure, function and blood flow. A major advantage of MR imaging is its exceptional spatial resolution. This coupled with a contrast agent that specifically homes to angiogenic vasculature, as provided in the present invention, will demonstrate angiogenic efficacy of angiogenic growth factor therapies. It will also make it possible to detect rupture prone atherosclerotic plaques in the vasculature, thereby identifying patients at high risk for myocardial infarction and cardiac death when these plaques are detected in the coronary vasculature. Recently, targeted MR imaging of tumour angiogenesis has been shown to be feasible with a liposome encapsulating a MR contrast molecule, targeted to the angiogenic vasculature with an antibody against the integrin α_{ν}β₃ (Sipkins et al., 1998, Nature Med. 4: 623-626).

In an eleventh aspect, the invention, therefore, relates to a conjugate comprising a CD13/APN homing molecule that is conjugated with an imaging moiety. The CD13/APN homing molecule is a homing molecule as herein defined above and may be conjugated to the imaging moiety by any of the means as described herein above.

An imaging moiety may be any molecule or structure that is capable of being detected, either directly or indirectly, in an *in vivo* diagnostic imaging procedure. Imaging moieties may thus be moieties that emit or be caused to emit detectable radiation (e.g. by spin resonance excitation or fluorescence excitation), moieties that affect local electromagnetic fields (e.g. paramagnetic, superparamagnetic, ferrimagnetic or ferromagnetic species), moieties that absorb or scatter radiation energy (e.g. chromophores, particles which may contain liquids or gas, heavy elements or compounds thereof) and moieties that are capable of generating a detectable substance (e.g. gas microbubble generators). The imaging moiety preferably is a moiety that is suitable for detection by any diagnostic imaging modality known in the art, such as e.g. X-ray, MR, ultrasound, scintigraphy, PET, SPECT, electrical impedance, light or magnetometric imaging modalities, of which MR and ultrasound are preferred.

A wide range of materials detectable by diagnostic imaging modalities is known from the art (see e.g. U.S. 6,264,914) and the imaging moiety will be selected according to the imaging modality to be used. Thus e.g. for ultrasound imaging an echogenic material, or a material capable of generating an echogenic material will normally be selected, for X-ray imaging the imaging moiety will generally be or contain a heavy atom (e.g. of atomic weight 38 or above), and for MR imaging the imaging moiety will either be a non zero nuclear spin isotope (such as ₁₉ F) or a material having unpaired electron spins and hence paramagnetic, superparamagnetic, ferrimagnetic or ferromagnetic properties. A preferred imaging moiety is not or does not contain a radioisotope, i.e. a preferred imaging moiety is not radioactive.

Examples of suitable imaging moieties are widely known from the diagnostic imaging literature, such as e.g. magnetic iron oxide particles, gas-containing vesicles and chelated paramagnetic metals (such as Gd, Dy, Mn, and Fe). See e.g. U.S. 4,647,447, WO 97/25073, U.S. 4,863,715, U.S. 4,770,183, WO 96/09840, WO 85/02772, WO 92/17212, WO 97/29783, EP-A-0554213, U.S. 5,228,446, WO 91/15243, WO 93/05818, WO 96/23524, WO 96/17628, U.S. 5,387,080, and WO 95/26205. Preferred imaging moieties are further discussed herein below.

A twelfth aspect of the invention relates to a composition, preferably a pharmaceutical composition comprising the conjugates of the CD13/APN homing molecule and the imaging moiety. The composition may comprise one such conjugate or may comprise combinations of different imaging conjugates that may be used for different imaging modalities. The pharmaceutical composition comprises at least one purified conjugate of the CD13/APN homing molecule and the imaging moiety and a pharmaceutical carrier. The pharmaceutical composition is further as herein described below. A preferred pharmaceutical composition is suitable for parenteral, or more preferably intravenous or intraarterial administration.

In a thirteenth aspect the invention relates to a method for vascular imaging, wherein the method comprises the steps of (1) administering a pharmaceutical composition comprising the conjugate of the CD13/APN homing molecule and the imaging moiety as defined above, to a subject requiring imaging of its vasculature; and (2) imaging the vasculature of the subject, preferably the angiogenic vasculature of the subject is imaged. The method preferably is a diagnostic method. Imaging may be by any of the imaging modalities described above, of which MR and ultrasound imaging are preferred.

A preferred method, is a method for imaging myocardial vasculature in a subject suffering from myocardial ischemia. Preferably, the angiogenesis in the myocardial vasculature is imaged. The method is preferably used to diagnose the angiogenic efficacy of an angiogenesis-promoting therapy such as discussed above. These diagnostic methods may advantageously be applied to a subject suffering from chronic myocardial ischemia.

In a further preferred method, the subject is suspected of having an atherosclerotic plaque, more preferably the subject is suspected of having a rupture prone atherosclerotic plaque. Thus, a preferred method is a diagnostic method for diagnosing the presence of atherosclerotic plaques, more preferably the presence of rupture prone atherosclerotic plaques in a subject suspected of having such plaques. In a further embodiment the diagnostic method is used to diagnose or to follow the status, size, risk of rupture or development of the atherosclerotic plaque over time, preferably in response to a therapeutic treatment of the atherosclerotic plaque, such as the treatments of atherosclerotic plaques as discussed above.

Another preferred method relates to imaging of angiogenesis in extremities in patients suffering from limb ischemia or ischemia of the upper extremities. The method is preferably used to diagnose the angiogenic efficacy of an angiogenesis-promoting therapy such as discussed above. These diagnostic methods may advantageously be applied to a subject suffering from ischemia of the extremities.
Preferably, in any of the above methods the pharmaceutical composition comprising the conjugates of the invention is administered via an intravenous or intraarterial route. In any of these methods, the pharmaceutical composition may be administered by (continuous) infusion or by bolus injection(s), or by combinations thereof. Preferably, in any of the above diagnostic methods (the pharmaceutical composition comprising) the conjugate of the CD13/APN homing molecule and the imaging moiety as defined above is administered in a diagnostically effective amount, preferably such that the stated indications can be diagnosed. The dose at which the vasculature can be imaged is anticipated to be lower than the dose for systemic, non-targeted administration because of the target specificity of NGR/imaging conjugates that home to CD13/APN.

In a fourteenth aspect the invention relates to use of a conjugate of the CD13/APN homing molecule and the imaging moiety as defined above, in the manufacture of a medicament or a pharmaceutical composition for use in any of the diagnostic imaging methods described above. The pharmaceutical composition preferably is suitable for administration via an intravenous or intraarterial route.

### Recombinant techniques and methods for recombinant production of (poly)peptides

Peptides and polypeptides for use in the present invention, such e.g. angiogenic growth factors and conjugates, can be prepared using recombinant techniques in which a nucleotide sequence encoding the polypeptide of interest is expressed in cultured cells such as described in Ausubel et al., "Current Protocols in Molecular Biology", Greene Publishing and Wiley-Interscience, New York (1987) and in Sambrook and Russell (2001) "Molecular Cloning: A Laboratory Manual (3^{rd} edition), Cold Spring Harbor Laboratory, Cold Spring Harbor Laboratory Press, New York; both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34:315 (describing cassette mutagenesis).

Typically, nucleic acids encoding the desired polypeptides are used in expression vectors. The phrase "expression vector" generally refers to nucleotide sequences that are capable of affecting expression of a gene in hosts compatible with such sequences. These expression vectors typically include at least suitable promoter sequences and optionally, transcription termination signals. Additional factors necessary or helpful in effecting expression can also be used as described herein. DNA encoding a polypeptide is incorporated into DNA constructs capable of introduction into and expression in an *in vitro* cell culture. Specifically, DNA constructs are suitable for replication in a prokaryotic host, such as bacteria, *e.g., E. coli*, or can be introduced into a cultured mammalian, plant, insect, e.g., Sf9, yeast, fungi or other eukaryotic cell lines.

DNA constructs prepared for introduction into a particular host typically include a replication system recognised by the host, the intended DNA segment encoding the desired polypeptide, and transcriptional and translational initiation and termination regulatory sequences operably linked to the polypeptide encoding segment. A DNA segment is "operably linked" when it is placed into a functional relationship with another DNA segment. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

The selection of an appropriate promoter sequence generally depends upon the host cell selected for the expression of the DNA segment. Examples of suitable promoter sequences include prokaryotic, and eukaryotic promoters well-known in the art (see, e.g. Sambrook and Russell, 2001, supra). The transcriptional regulatory sequences typically include a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, supra). Expression vectors include the replication system and transcriptional and translational regulatory sequences together with the insertion site for the polypeptide encoding segment can be employed. Examples of workable combinations of cell lines and expression vectors are described in Sambrook and Russell (2001, supra) and in Metzger et al. (1988) Nature 334: 31-36. For example, suitable expression vectors can be expressed in, *e.g.,* insect cells, *e.g.,* Sf9 cells, mammalian cells, *e.g.,* CHO cells and bacterial cells, *e.g., E. coli*.

*In vitro* mutagenesis and expression of mutant proteins are described generally in Ausubel et al. (1987, supra) and in Sambrook and Russell (2001, supra). Also see, Kunkel (1985, supra; describing site directed mutagenesis) and Roberts et al. (1987, supra; describing cassette mutagenesis).

Another method for preparing polypeptides is to employ an *in vitro* transcription/translation system. DNA encoding a polypeptide is cloned into an expression vector as described *supra.* The expression vector is then transcribed and translated *in vitro.* The translation product can be used directly or first purified. Polypeptides resulting from *in vitro* translation typically do not contain the post-translation modifications present on polypeptides synthesised *in vivo.* Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987 (incorporated herein by reference in its entirety)

### Pharmaceutical compositions

In the therapeutic and diagnostic methods of the invention, the conjugates with the CD13/APN homing molecule is administered in purified form together with a pharmaceutical carrier as a pharmaceutical composition. The preferred form depends on the intended mode of administration and therapeutic or diagnostic application. The pharmaceutical carrier can be any compatible, nontoxic substance suitable to deliver the conjugates to the patient. Pharmaceutically acceptable carriers are well known in the art and include, for example, aqueous solutions such as (sterile) water or physiologically buffered saline or other solvents or vehicles such as glycols, glycerol, oils such as olive oil or injectable organic esters, alcohol, fats, waxes, and inert solids may be used as the carrier. A pharmaceutically acceptable carrier may further contain physiologically acceptable compounds that act, e.g. to stabilise or to increase the absorption of the conjugate. Such physiologically acceptable compounds include, for example, carbohydrates, such as glucose, sucrose or dextrans, antioxidants, such as ascorbic acid or glutathione, chelating agents, low molecular weight proteins or other stabilisers or excipients. One skilled in the art would know that the choice of a pharmaceutically acceptable carrier, including a physiologically acceptable compound, depends, for example, on the route of administration of the composition. Pharmaceutically acceptable adjuvants, buffering agents, dispersing agents, and the like, may also be incorporated into the pharmaceutical compositions.

For oral administration, the active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medicatiorr over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance.

The conjugates are however preferably administered parentally. Preparations of the conjugates for parental administration must be sterile. Sterilisation is readily accomplished by filtration through sterile filtration membranes, optionally prior to or following lyophilisation and reconstitution. The parental route for administration of conjugates is in accord with known methods, e.g. injection or infusion by intravenous, intraperitoneal, intramuscular, intraarterial or intralesional routes. The conjugates may be administered continuously by infusion or by bolus injection. A typical composition for intravenous infusion could be made up to contain 100 to 500 ml of sterile 0.9% NaCl or 5% glucose optionally supplemented with a 20% albumin solution and 1 mg to 10 g of the conjugate, depending on the type of conjugate and its required dosing regime. Methods for preparing parenterally administrable compositions are well known in the art and described in more detail in various sources, including, for example, Remington's Pharmaceutical Science (15th ed., Mack Publishing, Easton, PA, 1980) (incorporated by reference in its entirety for all purposes).

The pharmaceutical composition also can be a CD13/APN homing molecule linked to liposomes or other polymer matrices, which can have incorporated therein, for example, an angiogenesis-promoting molecule, an angiogenesis inhibitor or an imaging moiety (Gregoriadis, Liposome Technology, Vol. 1 (CRC Press, Boca Raton, Fla. 1984), which is incorporated herein by reference). Liposomes, for example, which consist of phospholipids or other lipids, are nontoxic, physiologically acceptable and metabolisable carriers that are relatively simple to make and administer.

For the diagnostic or therapeutic methods disclosed herein, an effective amount of the CD13/APN homing molecule conjugate must be administered to the subject. As used herein, the term "effective amount" means the amount of the conjugate that produces the desired effect. An effective amount often will depend on the moiety linked to the CD13/APN homing molecule. An effective amount of a particular molecule/moiety for a specific purpose can be determined using methods well known to those in the art. The dose at which therapeutic effects can be achieved and the vasculature can be imaged is anticipated to be lower than the dose for systemic, non-targeted administration because of the target specificity of NGR/angiogenic and NGR/ imaging conjugates that home to CD13/APN.

### Imaging moieties

Particularly preferred as imaging moieties are: (1) chelated paramagnetic metal ions such as Gd, Dy, Fe, and Mn, especially when chelated by macrocyclic chelant groups (e.g. tetraazacyclododecane chelants such as DOTA, D03A, HP-DO3A and analogues thereof) or by linker chelant groups such as DTPA, DTPA-BMA, EDTA, and DPDP; superparamagnetic iron oxide crystals; (1) vesicles containing fluorinated gases (i.e. containing materials in the gas phase at 37° C. which are fluorine containing, e.g. SF₆ or perfluorinated C₁₋₆ hydrocarbons or other gases and gas precursors listed in WO 97/29783); chelated heavy metal cluster ions (e.g. W or Mo polyoxoanions or the sulphur or mixed oxygen/sulphur analogs); (3) covalently bonded non-metal atoms which are either high atomic number (e.g. iodine); and (4) iodinated compound containing vesicles.

Preferred paramagnetic metal ions include ions of transition and lanthanide metals (e.g. metals having atomic numbers of 6 to 9, 21-29, 42, 43, 44, or 57-71), in particular ions of Cr, V, Mn, Fe, Co, Ni, Cu, La, Ce, Pr, Nd, Pm, Sm, Eu, Gd, Tb, Dy, Ho, Er, Tm, Yb and Lu, especially of Mn, Cr, Fe, Gd and Dy, more especially Gd. Preferred heavy metal-containing imaging moieties may include atoms of Mo, Bi, Si, and W, and in particular may be polyatomic cluster ions (e.g. Bi compounds and W and Mo oxides) as described in WO 91/14460, WO 92/17215, WO 96/40287, and WO 96/22914. The metal ions are desirably chelated by chelant groups in the imaging moiety or in or on an imaging particle, (e.g. a vesicle or a porous or non-porous inorganic or organic solid), in particular linear, macrocyclic, terpyridine and N₂S₂ chelants, such as for example DTPA, DTPA-BMA, EDTA, D03A, TMT. Further examples of suitable chelant groups are disclosed in U.S. 4,647,447, WO 89/00557, U.S. 5,367,080, and U.S. 5,364,613. The imaging moiety or particle may contain one or more such chelant groups, if desired metallated by more than one metal species (e.g. so as to provide imaging moieties detectable in different imaging modalities). Preferably, a polychelant (particulate) imaging moiety is used. As is well known, a chelating agent is a compound containing donor atoms that can combine by coordinate bonding with a metal atom to form a cyclic structure called a chelation complex or chelate. This class of compounds is described in the Kirk-Othmer Encyclopedia of Chemical Technology, Vol. 5, 339-368.

Preferred non-metal atomic imaging moieties include non zero nuclear spin atoms such as ₁₈F, and heavy atoms such as I. Such imaging moieties, preferably a plurality thereof, e.g. 2 to 200, may be covalently bonded to a linker backbone, either directly using conventional chemical synthesis techniques or via a supporting group, e.g. a triiodophenyl group. As with the metal chelants discussed above, such non-metal atomic imaging moieties may be directly linked to CD13/APN homing molecule or carried in or on a particle carrying CD13/APN homing molecules, e.g. in a vesicle (see WO 95/26205 and GB 9624918.0).

Particulate imaging moieties generally fall into two categories: (1) those where the particle comprises a matrix or shell which carries or contains the imaging moiety and (2) those where the particle matrix is itself the imaging moiety. Examples of the first category are: vesicles (e.g. micelles, liposomes, microballoons and microbubbles) containing a liquid, gas or solid phase which contains the contrast effective imaging moiety, e.g. an echogenic gas or a precursor therefor (see for example GB 9700699.3), a chelated paramagnetic metal or a water-soluble iodinated X-ray contrast agent; porous particles loaded with the imaging moiety, e.g. paramagnetic metal loaded molecular sieve particles; and solid particles, e.g. of an inert biotolerable polymer, onto which the imaging moiety is bound or coated, e.g. dye-loaded polymer particles. Examples of the second category are: light scattering organic or inorganic particles; magnetic particles (ie. superparamagnetic, ferromagnetic or ferrimagnetic particles); and dye particles.

Preferred particulate imaging moieties include superparamagnetic particles (see U.S. 4,770,183, WO 97/25073, WO 96/09840, etc.), echogenic vesicles (see WO 92/17212, WO 97/29783, etc.), iodine-containing vesicles (see WO 95/26205 and GB 9624918.0), and dye-loaded polymer particles (see WO 96/23524). The particulate imaging moieties may have one or more CD13/APN homing molecules attached directly or indirectly to their surfaces. Generally it will be preferred to attach a plurality (e.g. 2 to 50) of CD13/APN homing molecule per particle. Particularly conveniently, besides the CD13/APN homing molecule, one will also attach flow decelerating molecules to the particles, i.e. molecules which have an affinity for the capillary lumen, which is sufficient to slow the passage of the contrast agent through the capillaries or the target organ but not sufficient on its own to immobilise the conjugate. Such flow decelerating vectors (described for example in GB 9700699.3) may moreover serve to anchor the conjugate once it has bound to its CD13/APN target site.

The means by which the CD13/APN homing molecule will be attached to the particle will depend on the nature of the particle surface. For inorganic particles, the conjugation to the particle may be for example by way of interaction between a metal binding group (e.g. a phosphate, phosphonate or oligo or polyphosphate group) on the CD13/APN homing molecule or on a linker attached to the CD13/APN homing molecule. For organic (e.g. polymeric) particles, CD13/APN homing molecule attachment may be by way of direct covalent bonding between groups on the particle surface and reactive groups in the CD13/APN homing molecule, e.g. amide or ester bonding, or by covalent attachment of CD13/APN homing molecule and particle to a (cross)linker. Generally, the means for conjugation of CD13/APN homing molecules and particles are as discussed above. However, for non-solid particles, e.g. droplets (for example of water insoluble iodinated liquids as described in U.S. 5,318,767, U.S. 5,451,393, U.S. 5,352,459 and U.S. 5,569,448) and vesicles, the CD13/APN homing molecule may be conjugated to a hydrophobic "anchor" groups, e.g. saturated or unsaturated C₁₂₋₃₀ chains, which will penetrate the particle surface and bind the CD13/APN homing molecule to the particle. Thus for phospholipid vesicles, the CD13/APN homing molecule may be bound covalently to a phospholipid compatible with the vesicle membrane. Examples of binding homing agents to vesicles and inorganic particles are described in GB 9622368.0 and WO 97/25073. Besides the vectors, other groups may be bound to the particle surface, e.g. stabilisers (to prevent aggregation) and biodistribution modifiers such as PEG. Such groups are discussed for example in WO 97/25073, WO 96/09840, EP-A-0284549 and U.S. 4,904,479.

Preferably the conjugates of the invention will have non-peptidic CD13/APN homing molecule coupled directly or indirectly to the imaging moiety, e.g. with covalently bound iodine radioisotopes, with metal chelates attached directly or via an organic linker group or coupled to a particulate imaging moiety, e.g. a superparamagnetic crystals (optionally coated, e.g. as in WO 97/25073), or a vesicle, e.g. a gas containing or iodinated contrast agent containing micelle, liposome or microballoon.

### Description of the figures

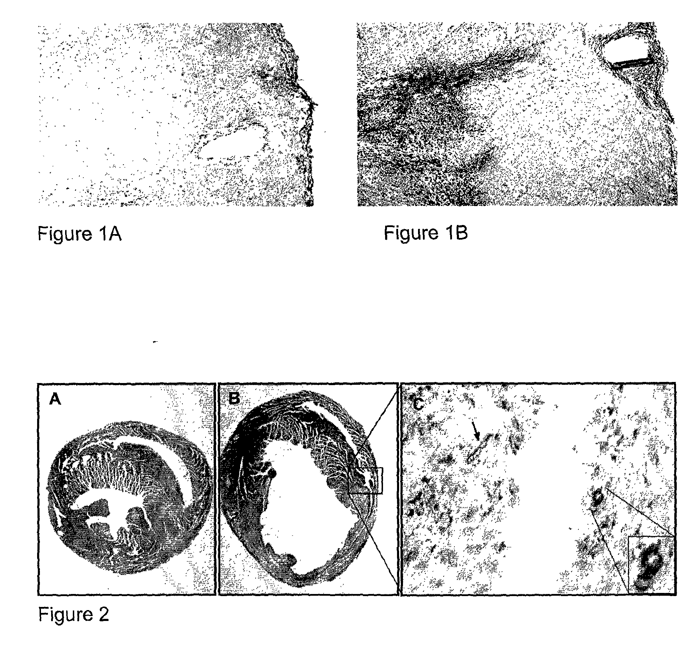

**Figure 1** CD13/APN stain in the ischemic border zone after murine MI (Figure 1B)), CD13/APN staining around LAD ligature is secondary to local inflammation. Non-infarcted area of the same heart (Figure 1A) showing no CD13/APN immunoreactivity.
**Figure 2. A:** No CD13/APN immuno-reativity in sham operated mice, **B**: murine infarct with left ventricular dilatation, scar formation and thinning of the left ventricular wall in the infarct zone, **C:** CD13/APN immuno-reactivity in capillaries of the peri-infarct zone, co-localising with the inflammatory mediator CD154 (in-set at higher magnification).

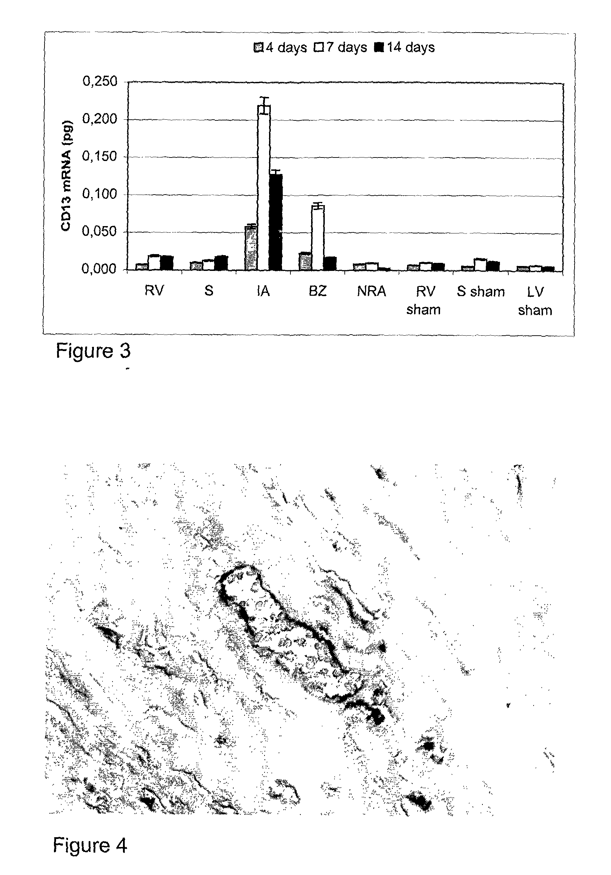

**Figure 3.** Time course of CD13/APN mRNA expression after murine myocardial infarction showing peak CD13/APN expression in the infarct area and the ischemic border zone at 7 days after infarction. BZ: border zone; IA: infarct area; LV sham: left ventricle in sham operated animals; NRA: non risk area; RV: right ventricle; RV sham; right ventricle in sham operated animals; S: septum; S sham: septum in sham operated animals.
**Figure 4.** CD13/APN immuno-reactivity in a capillary of a rupture prone human atherosclerotic plaque, identifying these capillaries as angiogenic vessels and targets for therapeutic agents or imaging molecules that, coupled to specific peptides, home to CD13/APN. The lumen of the capillary contains erythrocytes.

### Examples

### Example 1

### CD13/APN expression in angiogenic myocardial tissue.

### CD13/APN is upregulated by angiogenic growth factors in coronary endothelial cells.

Primary porcine coronary endothelial cells were incubated under serum free conditions for 24 hours before addition of medium containing either 1% serum, 25 ng/ml VEGF or 50 ng/ml bFGF. Cells were harvested after 24 hours, total RNA isolated, and semi-quantitative RT-PCR reactions were performed using primers common to both human and porcine CD13/APN. RT-PCR products were detected by ethidium bromide stain and Southern blot. CD13/APN induction levels (1.5-2.5 fold) were equivalent to levels reported previously in human umbilical vein endothelial cells. (Bhagwat et al., 2001, Blood 97:652-659).

### CD13/APN is upregulated in the ischemic border zone after murine myocardial infarction (MI).

Adult C57B1/J6 mice were subjected to ligation of the left anterior descending coronary artery, via left lateral thoracotomy under artificial ventilation and pentobarbital anaesthesia. Under ether anaesthesia, the mice were sacrificed 14 days after surgery. Following cardiac arrest in diastole by injection of 1 ml of cadmium chloride (0.1 M) into the inferior vena cava, animals were retrogradely perfused through the aorta with phosphate buffered saline (PBS; pH 7.4) containing 1 mg/ml sodium nitroprusside over 3 min of perfusion at a pressure of 100 mmHg, followed by 3 min perfusion with 5% formalin in PBS at the same pressure (Lutgens et al., 1999, Cardiovasc Res 41:586-593). The heart was dissected and fixed in 10% formalin in PBS for 2 hours and subsequently sectioned and stained anti-CD13/APN. The ischemic peri-infarct zone stained positive for CD13/APN, whereas non-infarcted myocardium of the same hearts showed no CD13/APN immuno-reactivity (see Figure 1). Moreover, CD13/APN immuno-reactivity was shown to be present in capillaries of the peri-infarct zone (see Figure 2), identifying these capillaries as angiogenic vessels and targets for therapeutic agents or imaging molecules that, coupled to specific peptides, home to CD13/APN.

### Example 2

### CD13/APN expression in rupture prone and ruptured human atherosclerotic plaques.

Vascular specimens were obtained from patients undergoing reconstructive surgery of the peripheral arterial system (aorta as well as iliac and femoral arteries). The specimens were formalin fixed and rupture-prone plaques as well as ruptured plaques were identified according to histo-morphologic criteria (Stary et al., 1995, Arterioscler Thromb Vasc Biol 15:1512-1531) and stained anti CD13/APN. Capillaries in these complex plaques showed CD13/APN immuno-reactivity (see Figure 3).

### Example 3

### Superiority of recombinant Placental Growth Factor (rP1GF) coupled to NGR in cardiac angiogenesis as compared to unconjugated rP1GF.

The present experiment compares the efficacy of an angiogenic growth factor (rP1GF) coupled to the homing sequence NGR and rP1GF alone with respect to enhancing angiogenesis in the murine peri-infarct zone. cDNA's coding for P1GF and P1GF with the peptide CNRGC fused to the P1GF C-terminus were expressed in mammalian cells in tissue culture by methods known in the art.

At the peak of CD13/APN expression after MI in P1GF -/- and wild type mice, the mice are divided into 3 groups receiving continuous growth factor delivery or placebo from an osmotic minipump (Alzet, Alza) for 7 days: group 1 receives 1.5 µg rP1GF/NGR per day, group 2 receives 1,5 µg rP1GF per day, group 3 receives placebo. Before sacrifice (after 7 days of treatment) in all animals the following parameters are examined: left ventricular pressures, stroke volume, cardiac output and wall motion (micromanometer tip pressure measurements + echocardiography). Myocardial perfusion is measured ex vivo in an isolated working mouse heart set up (n = 10 per treatment group). The capillary-to-muscle ratio and vascular density is measured and the formation of naked endothelial capillaries (angiogenesis) and muscularised arteries (arteriogenesis) are analysed (n = 10 per treatment group). Finally, in all animals, the accumulation of rP1GF/NGR is assessed immuno-histochemically in: brain, lung, liver, kidney and intestine. In previous studies (Heymans S , et al., 1999, Nat Med 5:1135-1142) 10 mice per group sufficed to detect a significant difference in angiogenesis between 2 treatment groups. Hence, the groups consists of 13 mice (3 extra mice to account for peri-operative mortality). The examined parameters show a statistically significant superiority of the conjugated P1GF in stimulating cardiac angiogenesis as compared to the non-conjugated P1GF.

### Example 4

### Superiority of the angiogenesis inhibitor endostatin coupled to NGR in atherosclerotic plaque stabilisation as compared to endostatin.

Mice lacking the ApoE gene and wild type mice are fed a diet containing 0.15% cholesterol from ages 6 weeks until 20 weeks. At 20 weeks of age 10 animals are killed to define the baseline extent of atherosclerosis. The other mice are divided into 3 groups receiving angiogenesis inhibitor or placebo for a period of 16 weeks via daily intra-peritoneal injection for a period of 16 weeks: group 1 receives 20mg/kg endostatin/NGR per day, group 2 receives 20mg/kg endostatin per day, group 3 receives placebo. After 16 weeks of treatment the mice are sacrificed under ketamine/xylazine anaesthesia and the vasculature is perfused via a catheter through the left ventricular apex for 3 min with 0.9% NaCl containing 20% nitroglycerin and perfusion fixed with 10% phosphate buffered formalin (pH 7.4) containing 20% nitroglycerin. Atherosclerotic lesions in the aortic sinus, the aortic arch and the descending aorta are classified according to lesion complexity (Stary et al., 1995, Arterioscler Thromb Vasc Biol 15:1512-1531) assessed for vascular density, infiltration of inflammatory cells, collagen content and size of the lipid core. Previous experiments (Moulton et al., 1999, Circulation 99:1726-1732) have shown that 12 mice per group were sufficient to detect a significant difference in plaque vascularisation and morphology. Hence, each group consists of 12 mice. The examined parameters show a statistically significant superiority of the conjugated endostatin in plaque stabilisation as compared to the non-conjugated endostatin.

## Claims

1. A conjugate comprising a CD13/APN homing molecule that is conjugated with a moiety for treating or diagnosing a vascular disease.

2. A conjugate according to claim 1 wherein the moiety is an angiogenesis-promoting molecule, and wherein the CD13/APN homing molecule preferably is a CD13/APN homing peptide.

3. A conjugate according to claim 2, wherein the angiogenesis-promoting molecule is an angiogenic growth factor, and wherein the angiogenic growth factor preferably is a peptide.

4. A conjugate according to claim 3, wherein the angiogenic growth factors is selected from the group consisting of VEGF, P1GF, bFGF, the macrophage-derived peptides PR39 and PR11, CD154, the extracellular domain of CD154 in soluble form, PDGF, HGF and scatter factor.

5. A conjugate according to claim 4, wherein the angiogenic growth factor is a heterodimer of VEGF and P1GF.

6. A conjugate according to any one of claims 3 - 5, wherein the conjugate is a single polypeptide chain that comprises the amino acid sequence of a CD13/APN homing peptide and the amino acid sequence of the angiogenic growth factor.

7. A conjugate according to claim 1, wherein the moiety is a moiety that stabilises or reduces atherosclerotic plaques, and wherein preferably the CD13/APN homing molecule is a CD13/APN homing peptide.

8. A conjugate according to claim 7, wherein the moiety that stabilises or reduces atherosclerotic plaques is selected from the group consisting of an anti-angiogenic agent, an HMG-coreductase inhibitor, an e-NOS or i-NOS expression vector, an antioxidant, 5-methyltetrahydrofolate and a CD154-antibody.

9. A conjugate according to claim 8, wherein the moiety that stabilises or reduces atherosclerotic plaques is an anti-angiogenic peptide is selected from the group consisting of endostatin, bestatin, TNP-470, angiostatin, thrombospondin 1 and 2, and the kringle 5 domain fragment from plasminogen.

10. A conjugate according to claims 8 or 9, wherein the conjugate is a single polypeptide chain that comprises the amino acid sequence of a CD13/APN homing peptide and the amino acid sequence of the anti-angiogenic peptide.

11. A conjugate according to claim 8, wherein the moiety that stabilises or reduces atherosclerotic plaques is selected from the group consisting of suramin, a 3-(3-substituted-1,2,4-thiadiazol-5-yl)pyridazine, thalidomide, a cytochalasin, iso-indolinone, and derivatives thereof.

12. A conjugate according to claim 1, wherein the moiety is an imaging moiety, and wherein preferably the CD13/APN homing molecule is a CD13/APN homing peptide.

13. A conjugate according to claim 12, wherein the imaging moiety is detectable by magnetic resonance, ultrasound or both.

14. A pharmaceutical composition comprising a conjugates as defined in claim 1, and a pharmaceutically acceptable carrier.

15. A pharmaceutical composition according to claim 14, wherein the conjugate is a conjugate as defined in any one of claims 2 - 6.

16. A pharmaceutical composition according to claim 14, wherein the conjugate is a conjugate as defined in any one of claims 7 - 11.

17. A pharmaceutical composition according to claim 14, wherein the conjugate is a conjugate as defined in claims 12 or 13.

18. Use of a conjugate as defined in any of claims 2 - 6, in the manufacture of a pharmaceutical composition for use in a therapeutic method for treating ischemic myocardium or an ischemic extremity or improving recovery after percutaneous coronary or peripheral intervention.

19. A use according to claim 18, wherein the therapeutic method is a method for treating a chronic ischemic myocardium.

20. A use according to claim 18, wherein the therapeutic method is a method for treating an ischemic myocardium that is the result of an myocardial infarction.

21. A use according to any one of claims 18 - 20, wherein the treatment results in:
(a) improved perfusion of the ischemic myocardium;
(b) a reduction in size of an ischemic zone in the ischemic myocardium;
(c) a reduction of the amount of myocardial tissue loss in the ischemic myocardium;
(d) prevention of further tissue loss in the ischemic myocardium; or
(e) a combination of thereof.

22. A use according to claim 18, wherein the therapeutic method is a method for treating a chronic ischemic extremity.

23. A use according to claim 18, wherein the therapeutic method is a method for treating an ischemic extremity that is the result of an arterial occlusion.

24. A use according to any one of claims 18, 22 or 23, wherein the treatment results in:
(a) improved perfusion of the ischemic extremity;
(b) a reduction in size of an ischemic zone in the ischemic extremity;
(c) a reduction of the amount of tissue loss in the ischemic extremity;
(d) prevention of further tissue loss in the ischemic extremity; or
(e) a combination thereof.

25. A use according to claim 18, wherein the therapeutic method is a method for accelerating wound healing after percutaneous coronary or peripheral intervention, or a method for preventing or reducing neointimal thickening of treated arterial segments.

26. Use of a conjugate as defined in any of claims 7 - 11 in the manufacture of a pharmaceutical composition for use in a therapeutic method for treating atherosclerotic plaques.

27. A use according to claim 26, wherein the therapeutic method is a method for treating a rupture prone atherosclerotic plaque.

28. A use according to claim 27, wherein the treatment stabilises or prevents rupture of the rupture prone plaque.

29. A use according to any one of claims 26 - 28, wherein the treatment reduces the size of the atherosclerotic plaque.

30. Use of a conjugate as defined in claims 12 or 13, in the manufacture of a pharmaceutical composition for use in a diagnostic method comprising vascular imaging.

31. A use according to claim 30, wherein the method is a method for diagnosing the presence of atherosclerotic plaques, more preferably the presence of rupture prone atherosclerotic plaques in a subject suspected of having such plaques.

32. A use according to claim 30, wherein the method is a method for imaging the vasculature of atherosclerotic plaques, preferably the angiogenic vasculature of such plaques.

33. A use according to claim 32, wherein the method is a method for diagnosing the status, size, risk of rupture or development of the atherosclerotic plaque in time, preferably in response to a therapeutic treatment of the atherosclerotic plaque.

34. A use according to claim 30, wherein the method is a method for imaging myocardial vasculature in a subject having suffered a myocardial infarction, whereby, preferably, angiogenesis in the myocardial vasculature is imaged.

35. A use according to claim 34, wherein the method is a method for imaging angiogenesis in the ischemic border zone at the perimeter of the infarcted myocardium, whereby, preferably the angiogenesis in response to an angiogenesis-promoting therapy is imaged to diagnose the efficacy the therapy.

36. A use according to claim 30, wherein the method is a method for imaging myocardial vasculature in a subject suffering from chronic myocardial ischemia.

37. A use according to claim 30, wherein the method is a method for imaging the vasculature of the extremities in a subject suffering from chronic ischemia of an extremity, whereby, preferably, angiogenesis in the extremity is imaged.

38. A use according to claim 37, wherein the method is a method for imaging the vasculature of the extremities in a subject having suffered from an acute or chronic arterial occlusion in an extremity, whereby, preferably, angiogenesis in the extremity is imaged.

39. A use according to claims 37 or 38, wherein the method is a method for imaging angiogenesis in the ischemic extremity, whereby, preferably the angiogenesis in response to an angiogenesis-promoting therapy is imaged to diagnose the efficacy of the therapy.

40. A use according to any one of claims 30 - 39, wherein the imaging is magnetic resonance or ultrasound imaging.


SEQUENCE LISTING

<110> Cardiovascular Research Institute Maastricht
      St. Jude Children's Research Hospital

<120> Targeting of myocardial angiogenesis through CD13/APN

<130> CD13/APN

<140> BO 44763
<141> 2001-11-11

<160> 5

<170> PatentIn Ver. 2.1

<210> 1
<211> 967
<212> PRT
<213> Homo sapiens

<400> 1
Met Ala Lys Gly Phe Tyr Ile Ser Lys Ser Leu Gly Ile Leu Gly Ile
  1               5                  10                  15

Leu Leu Gly Val Ala Ala Val Cys Thr Ile Ile Ala Leu Ser Val Val
             20                  25                  30

Tyr Ser Gln Glu Lys Asn Lys Asn Ala Asn Ser Ser Pro Val Ala Ser
         35                  40                  45

Thr Thr Pro Ser Ala Ser Ala Thr Thr Asn Pro Ala Ser Ala Thr Thr
     50                  55                  60

Leu Asp Gln Ser Lys Ala Trp Asn Arg Tyr Arg Leu Pro Asn Thr Leu
 65                  70                  75                  80

Lys Pro Asp Ser Tyr Gln Val Thr Leu Arg Pro Tyr Leu Thr Pro Asn
                 85                  90                  95

Asp Arg Gly Leu Tyr Val Phe Lys Gly Ser Ser Thr Val Arg Phe Thr
            100                 105                 110

Cys Lys Glu Ala Thr Asp Val Ile Ile Ile His Ser Lys Lys Leu Asn
        115                 120                 125

Tyr Thr Leu Ser Gln Gly His Arg Val Val Leu Arg Gly Val Gly Gly
    130                 135                 140

Ser Gln Pro Pro Asp Ile Asp Lys Thr Glu Leu Val Glu Pro Thr Glu
145                 150                 155                 160

Tyr Leu Val Val His Leu Lys Gly Ser Leu Val Lys Asp Ser Gln Tyr
                165                 170                 175

Glu Met Asp Ser Glu Phe Glu Gly Glu Leu Ala Asp Asp Leu Ala Gly
            180                 185                 190

Phe Tyr Arg Ser Glu Tyr Met Glu Gly Asn Val Arg Lys Val Val Ala
        195                 200                 205

Thr Thr Gln Met Gln Ala Ala Asp Ala Arg Lys Ser Phe Pro Cys Phe
    210                 215                 220

Asp Glu Pro Ala Met Lys Ala Glu Phe Asn Ile Thr Leu Ile His Pro
225                 230                 235                 240

Lys Asp Leu Thr Ala Leu Ser Asn Met Leu Pro Lys Gly Pro Ser Thr
                245                 250                 255

Pro Leu Pro Glu Asp Pro Asn Trp Asn Val Thr Glu Phe His Thr Thr
            260                 265                 270

Pro Lys Met Ser Thr Tyr Leu Leu Ala Phe Ile Val Ser Glu Phe Asp
        275                 280                 285

Tyr Val Glu Lys Gln Ala Ser Asn Gly Val Leu Ile Arg Ile Trp Ala
    290                 295                 300

Arg Pro Ser Ala Ile Ala Ala Gly His Gly Asp Tyr Ala Leu Asn Val
305                 310                 315                 320

Thr Gly Pro Ile Leu Asn Phe Phe Ala Gly His Tyr Asp Thr Pro Tyr
                325                 330                 335

Pro Leu Pro Lys Ser Asp Gln Ile Gly Leu Pro Asp Phe Asn Ala Gly
            340                 345                 350

Ala Met Glu Asn Trp Gly Leu Val Thr Tyr Arg Glu Asn Ser Leu Leu
        355                 360                 365

Phe Asp Pro Leu Ser Ser Ser Ser Ser Asn Lys Glu Arg Val Val Thr
    370                 375                 380

Val Ile Ala His Glu Leu Ala His Gln Trp Phe Gly Asn Leu Val Thr
385                 390                 395                 400

Ile Glu Trp Trp Asn Asp Leu Trp Leu Asn Glu Gly Phe Ala Ser Tyr
                405                 410                 415

Val Glu Tyr Leu Gly Ala Asp Tyr Ala Glu Pro Thr Trp Asn Leu Lys
            420                 425                 430

Asp Leu Met Val Leu Asn Asp Val Tyr Arg Val Met Ala Val Asp Ala
        435                 440                 445

Leu Ala Ser Ser His Pro Leu Ser Thr Pro Ala Ser Glu Ile Asn Thr
    450                 455                 460

Pro Ala Gln Ile Ser Glu Leu Phe Asp Ala Ile Ser Tyr Ser Lys Gly
465                 470                 475                 480

Ala Ser Val Leu Arg Met Leu Ser Ser Phe Leu Ser Glu Asp Val Phe
                485                 490                 495

Lys Gln Gly Leu Ala Ser Tyr Leu His Thr Phe Ala Tyr Gln Asn Thr
            500                 505                 510

Ile Tyr Leu Asn Leu Trp Asp His Leu Gln Glu Ala Val Asn Asn Arg
        515                 520                 525

Ser Ile Gln Leu Pro Thr Thr Val Arg Asp Ile Met Asn Arg Trp Thr

530                 535                 540

Leu Gln Met Gly Phe Pro Val Ile Thr Val Asp Thr Ser Thr Gly Thr
545                 550                 555                 560

Leu Ser Gln Glu His Phe Leu Leu Asp Pro Asp Ser Asn Val Thr Arg
                565                 570                 575

Pro Ser Glu Phe Asn Tyr Val Trp Ile Val Pro Ile Thr Ser Ile Arg
            580                 585                 590

Asp Gly Arg Gln Gln Gln Asp Tyr Trp Leu Ile Asp Val Arg Ala Gln
        595                 600                 605

Asn Asp Leu Phe Ser Thr Ser Gly Asn Glu Trp Val Leu Leu Asn Leu
    610                 615                 620

Asn Val Thr Gly Tyr Tyr Arg Val Asn Tyr Asp Glu Glu Asn Trp Arg
625                 630                 635                 640

Lys Ile Gln Thr Gln Leu Gln Arg Asp His Ser Ala Ile Pro Val Ile
                645                 650                 655

Asn Arg Ala Gln Ile Ile Asn Asp Ala Phe Asn Leu Ala Ser Ala His
            660                 665                 670

Lys Val Pro Val Thr Leu Ala Leu Asn Asn Thr Leu Phe Leu Ile Glu
        675                 680                 685

Glu Arg Gln Tyr Met Pro Trp Glu Ala Ala Leu Ser Ser Leu Ser Tyr
    690                 695                 700

Phe Lys Leu Met Phe Asp Arg Ser Glu Val Tyr Gly Pro Met Lys Asn
705                 710                 715                 720

Tyr Leu Lys Lys Gln Val Thr Pro Leu Phe Ile His Phe Arg Asn Asn
                725                 730                 735

Thr Asn Asn Trp Arg Glu Ile Pro Glu Asn Leu Met Asp Gln Tyr Ser
            740                 745                 750

Glu Val Asn Ala Ile Ser Thr Ala Cys Ser Asn Gly Val Pro Glu Cys
        755                 760                 765

Glu Glu Met Val Ser Gly Leu Phe Lys Gln Trp Met Glu Asn Pro Asn
    770                 775                 780

Asn Asn Pro Ile His Pro Asn Leu Arg Ser Thr Val Tyr Cys Asn Ala
785                 790                 795                 800

Ile Ala Gln Gly Gly Glu Glu Glu Trp Asp Phe Ala Trp Glu Gln Phe
                805                 810                 815

Arg Asn Ala Thr Leu Val Asn Glu Ala Asp Lys Leu Arg Ala Ala Leu
            820                 825                 830

Ala Cys Ser Lys Glu Leu Trp Ile Leu Asn Arg Tyr Leu Ser Tyr Thr
        835                 840                 845

Leu Asn Pro Asp Leu Ile Arg Lys Gln Asp Ala Thr Ser Thr Ile Ile
    850                 855                 860

Ser Ile Thr Asn Asn Val Ile Gly Gln Gly Leu Val Trp Asp Phe Val
865                 870                 875                 880

Gln Ser Asn Trp Lys Lys Leu Phe Asn Asp Tyr Gly Gly Gly Ser Phe
                885                 890                 895

Ser Phe Ser Asn Leu Ile Gln Ala Val Thr Arg Arg Phe Ser Thr Glu
            900                 905                 910

Tyr Glu Leu Gln Gln Leu Glu Gln Phe Lys Lys Asp Asn Glu Glu Thr
        915                 920                 925

Gly Phe Gly Ser Gly Thr Arg Ala Leu Glu Gln Ala Leu Glu Lys Thr
    930                 935                 940

Lys Ala Asn Ile Lys Trp Val Lys Glu Asn Lys Glu Val Val Leu Gln
945                 950                 955                 960

Trp Phe Thr Glu Asn Ser Lys
                965


<210> 2
<211> 13
<212> PRT
<213> Artificial Sequence

<220>
<223> Description of Artificial Sequence: peptide with
      CD13/APN binding sequence

<400> 2
Cys Asn Gly Arg Cys Val Ser Gly Cys Ala Gly Arg Cys
  1               5                  10


<210> 3
<211> 6
<212> PRT
<213> Artificial Sequence

<220>
<223> Description of Artificial Sequence: peptide with
      CD13/APN binding sequence

<400> 3
Asn Gly Arg Ala His Ala
  1               5


<210> 4
<211> 9
<212> PRT
<213> Artificial Sequence

<220>
<223> Description of Artificial Sequence: peptide with
      CD13/APN binding sequence

<400> 4

Cys Val Leu Asn Gly Arg Met Glu Cys
  1               5

<210> 5
<211> 5
<212> PRT
<213> Artificial Sequence

<220>
<223> Description of Artificial Sequence: peptide with
      CD13/APN binding sequence

<400> 5
Cys Asn Gly Arg Cys
  1               5